# EUROPEAN PATENT APPLICATION

(11) **EP 3 984 541 A1**
(43) Date of publication of application: **20.04.2022**
(21) Application number: 20822198.6
(22) Date of filing: 18.05.2020
(51) Int. Cl.: A61K 31/7105, A61K 31/7088, C12Q 1/6883, A61P 3/10

(54) **PREPARATION METHOD FOR DIABETES EARLY WARNING AND/OR DIAGNOSTIC REAGENT KIT BASED ON HSA-MIR-320A, MEDICAMENT FOR PREVENTING DIABETES, SCREENING METHOD FOR MEDICAMENT, AND PREPARATION METHOD THEREFOR**

(30) Priority: 11.06.2019 CN 201910500477; 11.06.2019 CN 201910500630
(71) Applicant: Tongji Hospital Tongji Medical College Huazhong University of Science and Technology, Hubei 430030 (CN)
(72) Inventor: WANG, Daowen, Wuhan, Hubei 430030 (CN); CHEN, Chen, Wuhan, Hubei 430030 (CN); LI, Huaping, Wuhan, Hubei 430030 (CN); WANG, Feng, Wuhan, Hubei 430030 (CN); YIN, Zhongwei, Wuhan, Hubei 430030 (CN)
(74) Representative: Schmitt-Nilson Schraud Waibel Wohlfrom Patentanwälte Partnerschaft mbB
(86) International application number: PCT/CN2020/090794
(87) International publication number: WO 2020/248771

(57) **Abstract**

The present invention "a medicament for preventing and treating diabetes based on hsa-miR-320a, a screening method for the medicament, and a preparation method therefor" relates to the field of molecular medicine and the diagnosis, prevention and treatment of metabolic diseases.. The active ingredient of the medicament has hsamiR-320a as the medicinal target and expresses the hsa-miR-320a by means of binding, and/or capturing, and/or degrading, and/or reducing to provide the efficacy of diabetes prevention. The hsa-miR-320a is as represented by SEQ ID No.1. Moreover, also provided in the present invention are the screening method and the preparation method for the medicament. The employment of the medicament of the present invention significantly reduces the blood sugar concentration of diabetic, effectively alleviate the symptoms of diabetes complications of a test animal and allows the test animal to maintain healthy and stable blood sugar over a long time.

## Description

### Technical Field

The present invention belongs to field of molecular medicine and diagnosis, prevention and treatment of metabolic diseases, in particular relates to a method preparing kit for early warning and/or diagnosing diabetes and a drug for preventing and treating diabetes based on hsa-miR-320a and its screening method as well as preparation method thereof.

### Background Art

MicroRNA (miRNA, miR) is a newly discovered class of endogenous small non-coding single stranded RNA with a length about 22 nucleotides which is from endogenous hairpin structure transcript. MicroRNA regulate gene expression after transcription by specifically binding to 3'untranslated region (3'untranslated region, 3'UTR) of target mRNA to promote degradation of target mRNA or inhibit translation of target gene and reduce coding protein level. miRNA is associated with various diseases, including neurodegenerative diseases, heart diseases, kidney diseases and tumor, etc. Recent studies have found that miRNA plays an important role in regulation of glucose and lipid metabolism and insulin resistance. Diabetes mellitus (DM) is a major disease that seriously threatens human life and health, and its prevalence rate is about 4.4% to 17.9% globally at present, which brings a heavy burden to patients and society. According to epidemiological statistics, the number of diabetes patients in the world reached 370 million in 2011, 80% of which were in developing countries. A total of 4.6 million people died of diabetes in the world that year, and the medical cost of diabetes in the world of that year reached 465 billion US dollars. In China, prevalence rate of diabetes has increased significantly in past 30 years. In 2013, the latest epidemiological survey warned that prevalence rate of diabetes among Chinese adults was 11.6% (about 110 million), and prevalence rate of prediabetes was as high as 50.1% (about 490 million).

More than 90% of diabetes patients are type 2 diabetes mellitus, which is a severe disorder of glycolipid metabolism caused by insulin resistance and β-cell failure and eventually leads to heart cerebrovascular and kidney complications and even death. Previous studies have shown that insulin resistance is a disorder of glycolipid metabolism caused by interaction of various genetic and environmental factors, including defective insulin signaling pathway, abnormal expression of insulin target and cross action of other hormone systems, and imbalance of other metabolic pathways. However, its specific pathophysiological mechanism and molecular signaling network are far from being fully understood and elucidated. Previously, pharmacotherapy strategies for diabetes have been mainly based on correcting glycolipid metabolism disorders, including drugs that promote insulin secretion (sulfonylureas, glinides, DPP-4 inhibitors) and hypoglycemic drugs through other mechanisms (biguanides, TZDs, α -glycosidase inhibitors).Even if patients receive treatment including insulin injection, only 40% of patients have good blood glucose control, and the metabolic disorders caused by diabetes cannot be effectively corrected. Therefore, new treatment measures are urgently needed. On the other hand, etiology and risk factors of diabetes have not been fully elucidated, so it is necessary to find new risk factors and adopt more effective risk assessment methods.

As an important endogenous regulatory factor, miRNAs are widely involved in regulation of signal transduction pathways in animals and plants. Although regulatory mechanism of miRNA has not been fully elucidated, existing research data indicate that miRNA plays an important role in regulation of glycolipid metabolism, which can not only serve as a new diagnostic marker of diabetes, but also participate in regulation of occurrence and development of diabetic insulin resistance and its complications. A series of studies have found significant differences in miRNA expression profiles in peripheral blood of diabetic patients compared with control population. miRNA-375 is specifically expressed in langerhans' islet β cells and participates in regulating secretion of insulin through the PI3K/PDK1/PKB signal pathway, while miR-375 knockout mice showed a glycolipid metabolism disorder phenotype. High systemic or islet specific let-7b expression results in impaired glucose tolerance and reduced insulin secretion. miR-503 expression is increased in endothelial cells under high glucose and is involved in regulation of diabetic vascular function through mediated by Cdc25A and CCNE1.

RNA interference technology is a promising biotechnology with breakthrough clinical application. In just a few years from its inception, several products have entered clinical trials and achieved great success. As disclosed in Chinese invention patent 201210069179.7, in previous research work, applicant of the invention found that miR-320a has a predictive role in atherosclerotic diseases, and studied to confirm the therapeutic role of anti-miR-320a, an antisense sequence of miR-320a, in atherosclerotic diseases. However, relationship between miR-320a and diabetes has not been found in this field, and the existing technologies lack effective products or drugs in molecular diagnosis and molecular therapy of diabetes.

### Summary of the invention

Based on above gaps and requirements in this field, the present invention further confirms the role of miR-320a in risk prediction of diabetes disease based on original basic work, the role of miR-320a participating in regulation of glucose metabolism and development of diabetes, and the therapeutic role of anti-miR-320a in diabetes. The present invention provides a novel medicinal use of endogenous non-coding small RNA, more specifically involving microRNA-320a (hsa-miR-320a) and its antisense nucleotide sequence hsa-anti-miR-320a in risk evaluation, prevention and treatment of diabetes mellitus. The invention also relates to the construction of a recombinant adeno-associated virus recombinant (rAAV-miR-320a/rAAV-anti-miR-320a) and its preparation method thereof. More specifically, it's related to cloning of hsa-miR-320a and anti-sense sequence anti-hsa-miR-320a and packaging and preparation of recombinant adeno-associated virus containing hsa-miR-320a and anti-hsa-miR-320a, respectively, and use of the recombinant adeno-associated virus in pharmaco.

The technical solution of the present invention is as follows:
On the one hand, the present invention provides a biomarker for the diagnosing and/ or early warning diabetes. characterized in that, comprises sequence fragment containing hsa-miR-320a; the hsa-miR-320a is shown as SEQ ID No.1.

The biomarker comprises said hsa-miR-320a.

The biomarker is said hsa-miR-320a.

In the 2^{nd} aspect, this invention provides a kit for diagnosing and/or early warning diabetes, characterized in that, comprises reagent for quantitative detection of said biomarker.

The kit comprises reagent for quantitative detection of hsa-miR-320a.

The reagent for quantitative detection of hsa-miR-320a comprises a specific primer pairs for hsa-miR-320a.

The specific primer pairs is commercially-available MIRQ0000510-1-1. Said primer product is purchased from Guangzhou Ribo Biotechnology Company Limited.

The regents for quantitative detection of hsa-miR-320a also comprises reverse transcription reagents; and / or reverse transcription PCR reagents;
preferably, the reverse transcription reagent comprises: RT Primer Mix, 2×TS reaction buffer, RNase free H₂O, TS enzyme;
further preferably, the reverse transcription PCR reagent comprises: 2×SYBR Green Mix, RNase free H₂O.

The quantitative detection refers to fluorescent quantitative PCR detection;
most preferably, the kit comprises miR-320a RT-primers, miR-320a real-time PCR forward primers, miR-320areal-time PCR reverse primer, SYBR Green I, TS reaction enzyme, TS reaction buffer and DEPC ddH₂O.

In the 3^{rd} aspect, this invention provides Use of the biomarker in preparing reagent for diagnosing diabetes.

In the 4^{th} aspect, this invention provides a drug for preventing and treating diabetes, characterized in that, a pharmacodynamic component of the drug is taking hsa-miR-320a as drug target, and efficacy of preventing and treating diabetes is achieved by binding, and/or capturing, and/or degrading hsa-miR-320a, and/or down-regulating expression of hsa-miR-320a;The hsa-miR-320a is shown as SEQ ID NO.1.

The pharmacodynamic component of the drug comprises: substance that can degrade hsa-miR-320a and /or down-regulate expression of hsa-miR-320a;
preferably, the pharmacodynamic component of the drug is a substance that can down-regulate expression of the hsa-miR-320a.

The pharmacodynamic component of the drug comprises: sequence fragment anti-hsa-miR-320a that is antisense complementary to hsa-miR-320a;
preferably, the antisense complementary refers to that length of said anti-hsa-miR-320a reverse complementary to full-length or partial sequence of the hsa-miR-320a is a sequence fragment of 15-25 bases.

The pharmacodynamic component of the drug is a recombinant plasmid expressing the sequence fragment anti-hsa-miR-320a that is antisense complementary to hsa-miR-320a.

The pharmacodynamic component of the drug is a recombinant adeno-associated virus plasmid pAAV-D(+)-anti-hsa-miR-320a, by which expressed sequence fragment anti-hsa-miR-320a is antisense complementary to hsa-miR-320a;
more specifically, the pAAV-D(+)-anti-miR-320a is constructed by inserting fragment amplified through primer pairs with sequences shown as SEQ ID NO.4 and SEQ ID NO.5 into a adenovirus expression vector pAAV-D(+).

The drug also comprises pharmaceutically acceptable excipients and / or reagents for buffering, culturing and/ or amplifying the recombinant adeno-associated virus plasmid pAAV-D(+)-anti-miR-320a.

In the 5^{th} of aspect, this invention provides a method of screening drug for preventing and treating diabetes, characterized in that, detecting whether candidate substance can bind, capture and degrade hsa-miR-320a and / or down regulate expression; preferably, substance that can reduce expression level of hsa-mir-320a is screened.

In the 6^{th} of aspect, this invention provides a method preparing drug for preventing and treating diabetes, characterized in that, comprises: taking substance that degrade hsa-miR-320a or down-regulate expression of hsa-miR-320a; sequence fragment anti-hsa-mir-320a antisense complementary to hsa-mir-320a;
recombinant plasmid expressing a sequence fragment of anti-hsa-mir-320a antisense complementary to hsa-miR-320a; and / or,
a recombinant adeno-associated virus vector expressing a sequence fragment of anti-hsa-miR-320a antisense complementary to hsa-miR-320a as an active ingredient in antidiabetic drug.

The method also comprises: primer pairs expressing sequence fragment anti-hsa-miR-320a antisense complementary to hsa-miR-320a is inserted into expression vector to prepare recombinant plasmid stably expressing sequence fragment anti-hsa-miR-320a antisense complementary to hsa-miR-320a.

Primer pairs which can express the sequence fragment of anti-hsa-miR-320a antisense complementary to hsa-miR-320a are shown as SEQ ID NO.4 and SEQ ID NO.5; the expression vector is adeno-associated virus expression vector pAAV-D(+).

Inventor of this invention found by a lot of experiments that expression of hsa-miR-320a in peripheral blood of diabetic patients was significantly increased. Furthermore, the inventor designed and synthesized sequences that express hsa-miR-320a and are antisense complementary to anti-hsa-miR-320a respectively according to hsa-miR-320a base sequence. Said sequences are respectively inserted into eukaryotic expression vector pAAV-D (+) to successfully construct a recombinant plasmids pAAV-D (+)-miR-320a and pAAV-D (+) -anti-miR-320a. After that, the following three plasmids were added: 1) pXX2, pXX8 or pXX9, 2) phelper, 3) pAAV-D(+)-miR-320a or pAAV-D(+)-anti-miR-320a were transformed into 293 cells by calcium and phosphorus co-transfection method to package and prepare three serotypes of recombinant adeno-associated viruses (rAAV2, rAAV8 and rAAV9) expressing hsa-miR-320a and antisense complementary anti-hsa-miR-320a respectively. After purification, titer was determined by real-time PCR. Next, two packaged recombinant adeno-associated viruses of same serotype (rAAV-miR-320a and rAAV-anti-miR-320a) were injected into db/db mice via tail vein injection respectively. It was found that blood glucose was significantly regulated in db/db mice, and recombinant adeno-associated virus mediated expression of anti-hsa-miR-320a significantly improved elevated blood glucose and cardiac function in db/db mice. While hsa-miR-320a further aggravated hyperglycemia and heart function in db/db mice. These results further support the therapeutic effect of anti-hsa-miR-320a on diabetes mellitus and its complications.

Specifically, the 1^{st} purpose of the present invention is to provide expression of miR-320a in peripheral blood of diabetic patients. The results shows that expression of miR-320a in peripheral blood of diabetic patients was significantly increased, which suggests that miR-320a can be used as a biomarker for early diagnosing and predicting diabetes.

The second purpose of the present invention is to confirm the protective effect of anti-miR-320a on diabetes through experiments based on the first purpose, anti-miR-320a can be used as a medicine to treat diabetes and its complications.

In conclusion, hsa-miR-320a discovered in the present invention can be used as an effective biomarker to evaluate risk of diabetes and to diagnose diabetes. The hsa-miR-320a-based kit is a highly efficient and accurate product in field of diabetes diagnosis. The drug for preventing and treating diabetes based on hsa-miR-320a in the present invention provides an effective and safe drug for treating diabetes in field of molecular biology, and provides a new choice to treat diabetes for patients and medical staffs.

### Description of Drawings

Above and other purposes and features of the invention will become clear from the description given below in conjunction with drawings.

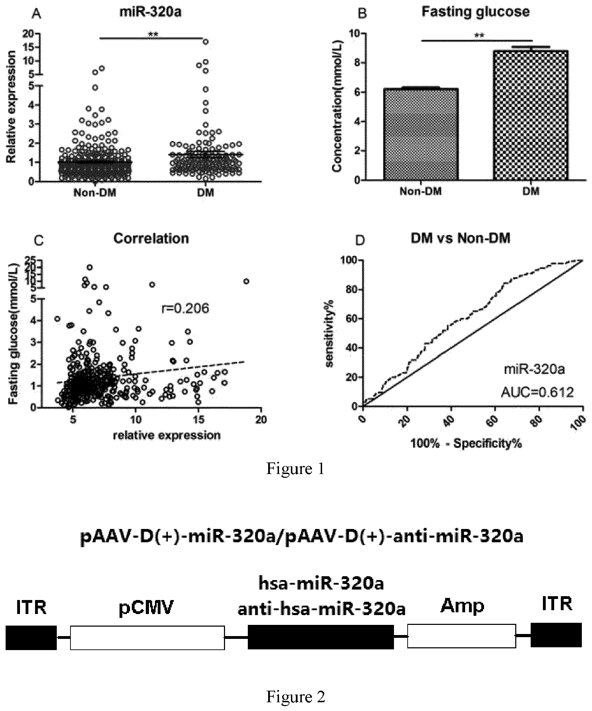

Figure 1 shows correlation between blood glucose level and hsa-miR-320a expression level in peripheral blood of diabetic patients. A. Expression of hsa-miR-320a in peripheral blood of diabetic patients was detected by real-time PCR; U6 was used as internal reference; method of 2⁻△△^{CT} is adopted as comparison method. B. value of fasting blood glucose in patients with diabetes. C. Correlation analysis of hsa-miR-320a expression and fasting blood glucose in peripheral blood. D. ROC curve of peripheral blood hsa-miR-320a for the diagnosis of diabetes mellitus; wherein, ^{∗∗}P<0.01; as compared with the control group (n=200). DM: diabetic patients; Non-DM: normal control people.
Figure 2 shows constitution of plasmids of pAAV-D(+)-miR-320a and pAAV-D(+)-anti-miR-320a.
Figure 3 shows that GFP was observed by fluorescence microscope (40X) after cell transfected by purified virus, the cells that were successfully transfected are shown in green with transfection efficiency more than 90%.
Figure 4 shows effects of microRNA treatments on fasting blood glucose in db/db mice. It's shown by fasting blood glucose detection that rAAV-miR-320a and rAAV-anti-miR-320a could significantly regulate the glycometabolism of db/db mice. (the former promotes increase of blood glucose, and the latter reduces blood glucose).
Figure 5 shows effects of microRNA treatments on cardiac ejection fraction in db/db mice. rAAV-anti-miR-320a significantly improve cardiac ejection fraction while rAAV-miR-320a reduces cardiac ejection fraction in db/db mice.

### Embodiments

The present invention will be further illustrated with the following specific examples. Advantages and characteristics of the present invention will become clearer with the description; however, these embodiments are only exemplary and do not limit the scope of the present invention. A person skilled in the art should understand that details and forms of the technical solution of the present invention may be modified or replaced without deviating from the conception and scope of the present invention, but such modifications and replacements fall within the protection scope of the present invention.

### Source of biological material

C57 control mice and db/db mice were purchased from Nanjing Model Animal Center (Nanjing, China). 293T cells were from China Center for Type Culture Collection in Wuhan University (Wuhan, China). Enzyme, plasmid, cells used in any of the following experiments and reagents, kit, consumable materials for routine use in various molecular biology experiments are commercially available.

The 1^{st} group of examples: The biomarker for diagnosing diabetes of the invention This group of examples provides a biomarker for the diagnosing and/ or early warning diabetes.

All embodiments in this group have following common features: the biomarker for the diagnosing and/ or early warning diabetes comprises sequence fragment containing hsa-miR-320a; the hsa-miR-320a is shown as SEQ ID No.1.

In specific embodiments, the biomarker comprises: said hsa-miR-320a.

In a more specific embodiment, the biomarker is the hsa-miR-320a.

The 2^{nd} group of examples: A kit for diagnosing/early warning/risk assessing diabetes of this invention

This group of examples provides a kit for diagnosing and/or early warning diabetes. All examples in this group have following common features: the kit for diagnosing and/or early warning diabetes comprises reagent for quantitative detection of said biomarker of any of the 1^{st} group of examples.

In further examples, the kit comprises reagent for quantitative detection of hsa-miR-320a.

In specific examples, the reagent for quantitative detection of hsa-miR-320a comprises a specific primer pairs for hsa-miR-320a.

In more specific examples, the specific primer pairs is commercially-available MIRQ0000510-1-1.

In preferred examples, the regents for quantitative detection of hsa-miR-320a also comprises reverse transcription reagents; and / or reverse transcription PCR reagents; preferably, the reverse transcription reagent comprises: RT Primer Mix, 2×TS reaction buffer, RNase free H₂O, TS enzyme;
further preferably, the reverse transcription PCR reagent comprises: 2×SYBR Green Mix, RNase free H₂O.

In some examples, the quantitative detection refers to fluorescent quantitative PCR detection;
In other preferred examples, the kit comprises miR-320a RT-primers, miR-320a real-time PCR forward primers, miR-320areal-time PCR reverse primer, SYBR Green I, TS reaction enzyme, TS reaction buffer and DEPC ddH₂O.

The 3^{rd} group of examples: Use of biomarker of this invention

This group of examples provides use of the biomarkers described in any of the 1^{st} group of examples in preparing reagents for diagnosing diabetes.

The 4^{th} group of examples: a drug for preventing and treating diabetes of this invention

This group of examples provides a drug for preventing and treating diabetes. All examples in this group have following common features: a pharmacodynamic component of the drug is taking hsa-miR-320a as drug target, and efficacy of preventing and treating diabetes is achieved by binding, and/or capturing, and/or degrading hsa-miR-320a, and/or down-regulating expression of hsa-miR-320a;The hsa-miR-320a is shown as SEQ ID NO.1.

In some examples, the pharmacodynamic component of the drug comprises: substance that can degrade hsa-miR-320a and /or down-regulate expression of hsa-miR-320a;
Preferably, the pharmacodynamic component of the drug is a substance that can down-regulate expression of the hsa-miR-320a.

In some other examples, the pharmacodynamic component of the drug comprises: sequence fragment anti-hsa-miR-320a that is antisense complementary to hsa-miR-320a;
In preferred examples, the antisense complementary refers to that length of said anti-hsa-miR-320a reverse complementary to full-length or partial sequence of the hsa-miR-320a is a sequence fragment of 15-25 bases.

In other preferred examples, the pharmacodynamic component of the drug is a recombinant plasmid expressing the sequence fragment anti-hsa-miR-320a that is antisense complementary to hsa-miR-320a.

In some specific examples, the pharmacodynamic component of the drug is a recombinant adeno-associated virus plasmid pAAV-D(+)-anti-hsa-miR-320a, by which expressed sequence fragment anti-hsa-miR-320a is antisense complementary to hsa-miR-320a;
In some more specific examples, the pAAV-D(+)-anti-miR-320a is constructed by inserting fragment amplified through primer pairs with sequences shown as SEQ ID NO.4 and SEQ ID NO.5 into a adenovirus expression vector pAAV-D(+).

In further examples, the drug also comprises pharmaceutically acceptable excipients and / or reagents for buffering, culturing and/ or amplifying the recombinant adeno-associated virus plasmid pAAV-D(+)-anti-miR-320a.

The 5^{th} group of examples: a method screening drug for preventing and treating diabetes of this invention

This group of examples provides a method screening drug for preventing and treating diabetes. All embodiments in this group have the following common features: detecting whether candidate substance can bind, capture and degrade hsa-miR-320a and / or down regulate expression;
In preferred examples, substance that can reduce expression level of hsa-mir-320a is screened.

The 6^{th} group of examples: a method preparing drug for preventing and treating diabetes of this inventionThis group of examples provides a method preparing drug for preventing and treating diabetes. All examples in this group have the following common features: said method comprises: taking substance that degrade hsa-miR-320a or down-regulate expression of hsa-miR-320a; sequence fragment anti-hsa-mir-320a antisense complementary to hsa-mir-320a;
recombinant plasmid expressing a sequence fragment of anti-hsa-mir-320a antisense complementary to hsa-miR-320a; and / or,
a recombinant adeno-associated virus vector expressing a sequence fragment of anti-hsa-miR-320a antisense complementary to hsa-miR-320a as an active ingredient in antidiabetic drug.

In further examples, the method also comprises: primer pairs expressing sequence fragment anti-hsa-miR-320a antisense complementary to hsa-miR-320a is inserted into expression vector to prepare recombinant plasmid stably expressing sequence fragment anti-hsa-miR-320a antisense complementary to hsa-miR-320a.

In specific examples, primer pairs which can express the sequence fragment of anti-hsa-miR-320a antisense complementary to hsa-miR-320a are shown as SEQ ID NO.4 and SEQ ID NO.5; the expression vector is adeno-associated virus expression vector pAAV-D(+).

Experimental example1: miRNA detection in peripheral blood of diabetic patients
1. Peripheral blood was collected from 200 diabetic patients and 200 healthy controls. The samples were centrifuged at 3500 RPM for 6 min at room temperature, and the upper plasma was collected and stored at -80°C. 1mL TRIZOL LS (Invitrogen, USA) was added into 0.25ml peripheral blood plasma. RNA was extracted using RNasey Mini Kit. The quality of RNA was detected by Nanodrop^{®} ND-1000.
2. Expression of hsa-miR-320a was detected by real-time PCR through using miRNA detection kit from Guangzhou Ribo Company .

miRNA reverse transcription:
RT Primer Mix:
   miRNA RT Primer 1 µl
   U6 RT Primer 1 µl
   RNase free H2O 78 µl
Reverse transcription reaction system:
   RNA template 2µg
   RT Primer Mix 4µl
   RNase free H2O up to 19 µl
Above systems were mixed, centrifuged instantaneously, incubated at 70°C for 10 min, ice incubated for 2 min; then the following reagents were added:
   2×TS reaction buffer 25µl
   TS enzyme 2.5 µl
   RNase free H2O 3.5 µl
Reverse transcription reaction procedure:
   42°C for 60min,70°C for 10min. Standby at 4°C after shutdown, and the product was stored at - 20 ° C.
miRNAs real-time PCR:
   Reaction system: 2×SYBR Green Mix 9 µl
   RT product 2µl
   miRNA Forward Primer 2µl(purchased from Guangzhou Ribo Biotechnology Company Limited)
   miRNA Reverse Primer 2µl(purchased from Guangzhou Ribo Biotechnology Company Limited)RNase-free H2O 5µl
reaction procedure:
   95°C 30sec-- (95°C 10sec--60°C 20sec --70°C 1sec) ×40 cycles--Melting Curve.

The results showed that expression level of hsa-miR-320a in peripheral blood of diabetic patients was increased (Figure 1A). Fasting blood glucose were also increased significantly (Figure IB). Expression level of hsa-miR-320a was positively correlated with fasting blood glucose level (Figure 1C). hsa-miR-320a could be used for diagnosing diabetes (Figure ID).
Sequence of hsa-miR-320a: aaaagcuggguugagagggcga (SEQ ID NO.1).

### Experimental example 2: Preparating kit to assess diabetes risk

The kit comprises the following main components: a set of specific reverse transcription primers and real-time PCR primer pairs used for amplifying miR-320a; a set of specific reverse transcription primers and real-time PCR primer pairs used to amplify control RNA (U6) and related reagents. Components and contents are as follows (100 times) and stored at - 20 °C:

| Components | Concentration | Volume | Source |
|---|---|---|---|
| miR-320a RT primer | 5umol/L | 50ul | Guangzhou Ribo Company |
| miR-320a real-time PCR forward primer | 5umol/L | 200ul | Guangzhou Ribo Company |
| miR-320a real-time PCR reverse primer | 5umol/L | 200ul | Guangzhou Ribo Company |
| U6 reverse transcription primer | 5umol/L | 50ul | Guangzhou Ribo Company |
| U6 real-time PCR forward primer | 5umol/L | 200ul | Guangzhou Ribo Company |
| U6 real-time PCR reverse primer | 5umol/L | 200ul | Guangzhou Ribo Company |
| SYBR Green I | 2 × | 1ml | Life Company |
| TS reaction enzyme | 2× | 150ul | Life Company |
| TS reaction buffer | | 1.5ml | Life Company |
| DEPC ddH₂O | | 1ml | - |

The above reagents are provided by various companies and have been commercialized. Specific detection methods and related reaction parameters refer to experimental example 1.

### Experimental example 3. Validation of accuracy of kit for early warning /diagnosing diabetes:

Currently, fasting blood glucose detection and OGTT test are commonly used to diagnose diabetes. Blood samples were collected from 100 patients who had been previously diagnosed with diabetes by above method. The biomarker provided by any of the 1^{st} group of examples and the kit for early warning /diagnosing diabetes provided by the 2^{nd} group of examples of the present invention are used for molecular detection. The diagnostic criteria for test are: The expression of miR-320a was higher than?? (currently no large-scale population data) is diagnosed as diabetes. The final result shows that there are ??, of which expression level of miR-320a in blood samples of diabetic patients is higher than?. Accuracy of the kit for early warning / diagnosing diabetes is about ?%. (there is no large-scale population data at present). Currently, fasting blood glucose detection and OGTT test are commonly used to diagnose diabetes. Blood samples were collected from 100 patients who had been previously diagnosed with diabetes by above method. The biomarker provided by any of the 1^{st} group of examples and the kit for early warning /diagnosing diabetes provided by the 2^{nd} group of examples of the present invention are used for molecular detection. The diagnostic criteria for test are: The expression of miR-320a was higher than that of 50nmol/L is diagnosed as diabetes. The final result shows that there are 83 patients of which expression level of miR-320a in blood samples of diabetic patients is higher than 50nmol/L. Accuracy of the kit for early warning / diagnosing diabetes is about 83%.

### Experimental example 4. Construction of recombinant adeno-associated virus

### 1. Synthesis of inserting sequence

Two reverse complementary strands of hsa-miR-320a and reverse complementary anti-hsa-miR-320a were designed and synthesized, respectively, based on the sequence of hsa-miR-320a (Figure 2)
Primer pairs expressing hsa-miR-320a:
   hsa-miR-320a-Sense:
      5' agctttcgccctctcaacccagcttttttcaagagaaaaagctgggttgagagggcgaccgc 3' ( SEQ ID NO.2)
   hsa-miR-320a-Antisense :
      3' aagcgggagagttgggtcgaaaaaagttctctttttcgacccaactctcccgctggcgccgg 5' ( SEQ ID NO.3)
Primer pairs expressing hsa-miR-320a:
   anti-hsa-miR-320a-Sense :
      5' agcttaaaagctgggttgagagggcgattcaagagatcgccctctcaacccagcttttccgc 3' (SEQ ID NO.4)
   anti-hsa-miR-320a-Antisense :
      3' attttcgacccaactctcccgctaagttctctagcgggagagttgggtcgaaaaggcgccgg 5' ( SEQ ID NO.5)

### 2. Experiments carried out according to the system and temperature in manual Nuclease-Free Water 36µl

Annealing Buffer for DNA Oligos (5×) 10µl
DNA oligo A (50µM) 2µl
DNA oligo B (50µM) 2µl
90°C for 3min, 37°C for 1hr, stored at 4°C_{∘}

### 3. Enzyme digestion reaction

Eukaryotic expression vector pAAV- D (+) was digested by BamH I and Not I at 37°C for 2 hr, digestion reaction system was as follows:
10×K Buffer 1µl
BSA 1 µl
BamH I 1 µl
Not I 1 µl
pAAV-D(+) 2 µl
ddH₂O 14 µl

### 4. Agarose Gel electrophoresis recovery

Double digestion products were separated by 1% Agarose Gel electrophoresis, and then recovered by TaKaRa Agarose Gel DNA Purification Kit Ver. 2.0. Detailed procedure is as follows:
1. 1xTAE buffer agarose gel was prepared and agarose gel electrophoresis was performed to target DNA;
2. Agarose gel containing the target DNA was cut out under ultraviolet light;
3. Weigh the gel block, calculate the volume of the gel block, cut up the gel block;
4. Add DR-I Buffer with volume being 3 times as many as gel block volume into the glue block, and heat the glue block to be melt at 75°C;
5. Add DR-II Buffer with volume being 1/2 as many as volume of DR-I Buffer to the glue block, and mix evenly; when separating DNA fragments less than 400 bp, isopropyl alcohol with a final concentration of 20% should be added to the solution.
6. Place the Spin Column on Collection Tube;
7. The solution in above step 5 was transferred to Spin Column, centrifuged at 12000 rpm for 1min and the filtrate was discarded;
8. 500µl Rinse A was added into the Spin Column, centrifuged at 12000 rpm for 30 sec, and the filtrate was discarded;
9. 700µl Rinse B was added into the Spin Column, centrifuged at 12000 rpm for 30 sec, and the filtrate was discarded;
10. Repeat above step 9;
11. The Spin Column was placed on the Collection Tube and centrifuged at 12000 RPM for Imin;
12. The Spin Column was placed on a new 1.5 mL centrifuge tube, and 25µL of 60°C preheated Elution Buffer was added to the center of the Spin Column membrane, and stood for 1min at room temperature;
13. Centrifuged at 12000 rpm for 1min to elute DNA.

### 5. Plasmid ligation

1. The pAAV-D(+) vector and the synthesized DNA fragments were ligated by T4 ligase and incubated overnight at 16°C as follows:
   10× T4 DNA ligase buffer 2.5 µl
   DNA 0.3 pmol
   Vector 0.03 pmol
   T4 DNA ligase 1 µl
   ddH₂O up to 25 µl
2. Add whole volume (25µl) to 100µl DH5α receptor cells and place in ice for 30min;
3. After heat for 45 sec at 42°C, and then place in ice for 1min;
4. 500µl antibiotic free LB medium was added, and the culture was shaken at 100rpm at 37 °C for 60min;
5. Culture on LB plate medium containing Amp ⁺, then white monoclonal colonies were selected.6. Plasmid Extraction at small scale

Select monoclonal colonies, add them to 3ml amp + LB liquid medium, and shake at 37 ° C 280rpm overnight. The plasmid was extracted using EasyPure Plasmid MiniPrep Kit purchased from Beijing TransGen Biotechnology Company Limited. The specific steps were as follows:
1. Take 1.5ml of overnight cultured bacteria, centrifuge at 10000g for 1min and suck up supernatant as much as possible;
2. Add 250 µL colorless solution RB (containing RNase A), shake to suspense bacterial precipitation;
3. Add 250 µL blue solution LB, gently turn up and down and mix for 4-6 times to fully lyse the bacteria to form a blue transparent solution;
4. Add 350 µL yellow solution NB, gently mix for 5-6 times until a compact yellow agglutination block formed, and stand at room temperature for 2min;
5. Centrifuge at 15000g for 5min, carefully absorb the supernatant and add it to the adsorption column;
6. Centrifuge at 15000g for 1min and discard the effluent;
7. Add 650 µL solution WB, centrifuge at 15000g for 1min and discard the effluent;
8. Centrifuge at 15000g for 2min to completely remove the residual WB;
9. Place the adsorption column in a new EP tube and add 20 µL EB preheated at 70 °C, standing at room temperature for 1 min;
10. Centrifuge at 10000g for Imin, elute DNA, and store the washed DNA at -20 °C.

### 7. Plasmid identification

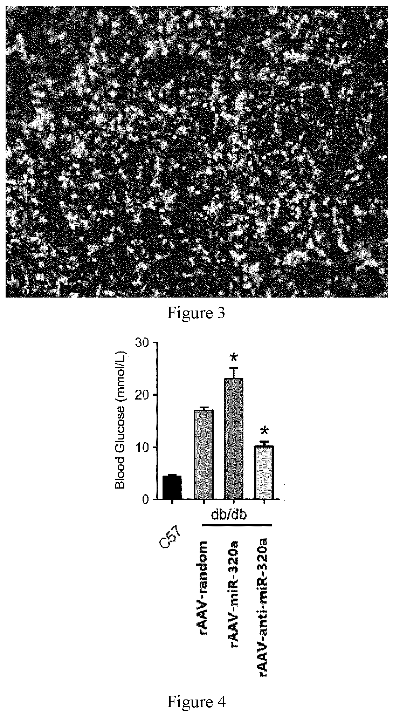

The constructed plasmids were identified by double enzyme digestion and sequencing, and the eukaryotic expression plasmids pAAV-D(+)-miR-320a and pAAV-D(+)-anti-miR-320a were obtained. Their structures are shown in Figure 3.

### 8. Plasmid Extraction at large scale

Prepare 1L sterile conical flask, add 300ml sterile LB medium, and add ampicillin solution to the final concentration of 100 µg/ml. Add 50µL of the required plasmid (pxx2, pxx8 or pxx9; phelper; pAAV-D(+), pAAV-D(+)-miR-320a or pAAV-D(+)-anti-miR-320a), 280 rpm, 37 °C overnight culture. Plasmids were extracted according to manual of E.Z.N.A.^{®} Endo-Free Plasmid Maxi Kit purchased from OMEGA company. The specific steps were as follows:
1. Collect bacteria by being centrifugede at 5000g at room temperature for 10min;
2. Discard the culture medium, add 10ml Solution I/RNase A mixture, vortex for oscillation and complete resuspension;
3. Add 10ml Solution II into the resuspended mixture, gently reverse and mix it for 10-15 times, and place it at room temperature for 2min;
4. Add 5ml Buffer N3 and gently reverse it several times to form white flocculent precipitation;
5. Put HiBind column in the collection pipe, add 5ml Buffer GPS, stand at room temperature for 3-10min, centrifuge at 5000g for 5min, discard the filtrate, and put the column back into the collection pipe;
6. Pour the bacterial lysate into the syringe filter, stand for 2min, insert and push the piston to collect the filtered lysate;
7. Add 1/10 volume of ETR into the filtered pyrolysis solution, reverse it for 7-10 times, and then ice bath for 10min;
8. After water bath at 42 °C for 5min, centrifuge at 5000g at room temperature for 5min, transfer the supernatant to a new centrifuge tube, add 0.5 times volume of absolute ethanol, mix well and stand at room temperature for 2min;
9. Transfer the mixed solution to the activated HiBind column, centrifuge at room temperature of 5000g for 5min, and discard the filtrate;
10. Reinstall the binding column into the collection pipe, add 10ml HB buffer, centrifuge at 5000g at room temperature for 5min, and discard the filtrate;
11. Reinstall the binding column into the collection pipe, add 15ml DNA wash buffer, centrifuge at 5000g at room temperature for 5min, and discard the filtrate;
12. Repeat above step;
13. Discard the filtrate, reinstall the binding column into the collection pipe, and centrifuge at 6000g for 15min;
14. Take out the binding column and dry it at 65 °C for 10min;
15. Put the binding column into a new centrifuge tube, add 1-3ml Endotoxin free Elusion Buffer preheated at 70 °C, stand at room temperature for 2min, and then centrifuge at 6000g for 5min to elute DNA.

### 9. rAAV mediated virus packaging

293T cells (human embryonic renal epithelial cells) grow to 90%. 1-2 hours before calcium and phosphorus transfection, change 12-15ml of fresh medium (including serum) for each Petri dish, first add calcium chloride (CaCl₂) into 50ml centrifuge tube, and then add plasmid to form Ca-DNA mixture, fully mix well, and slowly drop 2× HEBS BUFFER into Ca-DNA mixture to form Ca-DNA-P mixture, Shake the centrifuge tube while adding 2× HEBS to fully mix to form calcium and phosphorus particles. After 8-12 hours, change 18-20ml serum-free medium. After 72 hours, suck and discard the medium, wash it with PBS for 3 times, add 1ml Tris + NaCl (pH 8.5) to each Petri dish, curet the cells with a curette, collect them in a clean centrifuge tube and freeze at -80°C.

### 10. Virus purification

Take out the cells frozen at -80°C, thaw and dissolve at 37 °C, freeze and thaw repeatedly for 4 times, centrifuge at 8000g for 15min, put the supernatant into a clean centrifuge tube, and discard the cell precipitation.

Fully mix anhydrous ethanol precooled at -20 °C with rAAV in the volume ratio of 3:1. After being placed in the refrigerator at -20 °C for 2 hours, centrifuge at 4 °C by 13000 rpm for 15 minutes, and discard the supernatant; After ethanol volatilization, add corresponding volume of Tris + NaCl (pH 8.5) to dissolve the precipitation. Filter with millipore filter (0.22 µ m).

### 11. Virus titer determination

Sample treatment: rAAV virus solution 40 µl
   Protease K (20mg / ml) 5 µl
55 °C, reaction 1 hr;
   Phenol: chloroform: isoamyl alcohol 45 µl
4 °C, 12000g, centrifugation for 5min to recover the aqueous phase;
   Chloroform 45 µl
4 °C, centrifuged at 12000g for 5min to recover the aqueous phase.

Real-time PCR :
Primer 1(10µm) 0.4µl
Primer 2(10µm) 0.4µl
SYBR Green I Mix 10µl
ddH₂O 8.2µl
Template 1µl
95°C 30sec---(95°C 5sec---60°C 5sec ---72°C 20sec)×40 cycles ---Melting Curve

### 12. Virus transfection efficiency

After the purified virus was transfected into 293T cells for 48 hours, the proportion of transfected cells was observed by fluorescence microscope, and the transfection efficiency was more than 90% (Figure 4).

Experimental example 5. a recombinant adeno-associated virus of rAAV9 type expressing hsa-miR-320a/hsa-anti-miR-320a was used as an example to test its therapeutic effect on diabetes and complications.

### 1. Detection of blood glucose in db/db mice

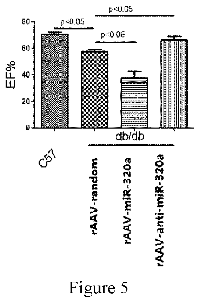

12 week old C57 control and db/db diabetic mice were used to inject rAAV-miR-320a and rAAV-anti-miR-320a through caudal vein injection respectively. Titer of virus was 1×10¹¹PFU. The fasting blood glucose of db/db mice was detected by taking caudal vein as sample to be tested through test paper at the end of the experiment (12 weeks later). The results showed that the blood glucose of db/db diabetic mice was significantly higher than that of C57 control mice. rAAV-miR-320a treatment significantly increased blood glucose, while rAAV-anti-miR-320a treatment significantly reduced blood glucose (Figure 5).

### 2. Cardiac function detection of db / db mice

The cardiac function of db/db mice was measured by cardiac ultrasound at the end of the experiment. The methods were as follows:
The instrument used is an ultrasonic instrument equipped with a 30MHz highfrequency probe. After the mice were anesthetized with isoflurane, the mice were placed on their back on detection platform, and two-dimensional images of the left ventricle were collected along the horizontal short axis and long axis of left ventricular papillary muscle near sternum of mice. At the same time, M-mode ultrasound images of more than 5 consecutive cardiac cycles were obtained respectively under guidance of two-dimensional images. According to the collected images, software was used to analyze results, and cardiac hemodynamic indexes detected by cardiac ultrasound were obtained. After analysis by relevant software, the following indexes were calculated: Heart Rate (HR); Left Ventricular Internal Dimension, diastole, LVIDd; Left Ventricular Internal Dimension, systole, LVIDs, Left Ventricular Posterior Wall, diastole, LVPWd; Left Ventricular Posterior Wall, systole, LVPWs; Interventricular septal thickness; diastole; IVSd; Interventricular septal thickness, systole, IVSs; Ejection Fraction, EF and Fractional Shortening, FS, and etc.The results showed that compared with C57 control mice, the cardiac systolic function of db/db mice was significantly impaired. rAAV-anti-miR-320a treatment could significantly improve the impaired cardiac function of db/db mice, while rAAV-miR-320a treatment could significantly aggravate the cardiac function of damaged db/db mice (Figure 6).

### Experimental example 6. animal clinical treatment verfication on drug preventing and treating diabetes of the invention

Hundreds of diabetic mice were treated with drug preventing and treating diabetes of this invention. Symptoms of each diabetic mouse before treatment include high blood sugar and diabetic complications, for example, impaired cardiac contractility. Treatment of diabetes was specifically as follows: the drug preventing and treating diabetes provided by any of the 4^{th} group of examples, and/or, drug preventing and treating diabetes screened by the method provided by any of the 5^{th} group of examples, and/or, drug preventing and treating diabetes prepared by the method provided by any of the 6^{th} group of examples were injected into db/db mice once through caudal vein injection at their age of 12 weeks. Virus titer was 1.0 × 10¹¹PFU / mouse, and blood glucose and cardiac contractility of each mouse were detected after 12 weeks. It was found that blood glucose and heart systolic function of all diabetic mice were significantly improved after the above treatment (comparison data charts before and after treatment in each mouse were similar to results shown in figures 4 and 5. In order to save space, this disclosure does not list them one by one). This shows that accuracy rate of animal clinical treatment for drug preventing and treating diabetes of this invention is 100%. At the same time, all mice injected with above drugs were followed up and observed half a year later. There were no abnormalities or other side effects. At the same time, blood glucose remained stable, and symptoms of complications did not appear again within half a year.

## Claims

1. A drug for preventing and treating diabetes, **characterized in that**, a pharmacodynamic component of the drug is taking hsa-miR-320a as drug target, and efficacy of preventing and treating diabetes is achieved by binding, and/or capturing, and/or degrading hsa-miR-320a, and/or down-regulating expression of hsa-miR-320a;The hsa-miR-320a is shown as SEQ ID NO.1.

2. The drug according to claim 1, **characterized in that**, the pharmacodynamic component of the drug comprises: substance that can degrade hsa-miR-320a and /or down-regulate expression of hsa-miR-320a;
preferably, the pharmacodynamic component of the drug is a substance that can down-regulate expression of the hsa-miR-320a.

3. The drug according to claim 1 or 2, **characterized in that**, the pharmacodynamic component of the drug comprises: sequence fragment anti-hsa-miR-320a that is antisense complementary to hsa-miR-320a;
preferably, the antisense complementary refers to that length of said anti-hsa-miR-320a reverse complementary to full-length or partial sequence of the hsa-miR-320a is a sequence fragment of 15-25 bases.

4. The drug according to claim 3, **characterized in that**, the pharmacodynamic component of the drug is a recombinant plasmid expressing the sequence fragment anti-hsa-miR-320a that is antisense complementary to hsa-miR-320a.

5. The drug according to claim 4, **characterized in that**, the pharmacodynamic component of the drug is a recombinant adeno-associated virus plasmid pAAV-D(+)-anti-hsa-miR-320a, by which expressed sequence fragment anti-hsa-miR-320a is antisense complementary to hsa-miR-320a;
more specifically, the pAAV-D(+)-anti-miR-320a is constructed by inserting fragment amplified through primer pairs with sequences shown as SEQ ID NO.4 and SEQ ID NO.5 into a adenovirus expression vector pAAV-D(+).

6. The drug according to any of claims 1-5, **characterized in that**, the drug also comprises pharmaceutically acceptable excipients and / or reagents for buffering, culturing and/ or amplifying the recombinant adeno-associated virus plasmid pAAV-D(+)-anti-miR-320a.

7. A method of screening drug for preventing and treating diabetes, characterized inthat, detecting whether candidate substance can bind, capture and degrade hsa-miR-320a and / or down regulate expression;
preferably, substance that can reduce expression level of hsa-mir-320a is screened.

8. A method preparing drug for preventing and treating diabetes, **characterized in that**, comprises: taking substance that degrade hsa-miR-320a or down-regulate expression of hsa-miR-320a; sequence fragment anti-hsa-mir-320a antisense complementary to hsa-mir-320a;
recombinant plasmid expressing a sequence fragment of anti-hsa-mir-320a antisense complementary to hsa-miR-320a; and / or,
a recombinant adeno-associated virus vector expressing a sequence fragment of anti-hsa-miR-320a antisense complementary to hsa-miR-320a as an active ingredient in antidiabetic drug.

9. The method according to claim 8, **characterized in that**, also comprises: primer pairs expressing sequence fragment anti-hsa-miR-320a antisense complementary to hsa-miR-320a is inserted into expression vector to prepare recombinant plasmid stably expressing sequence fragment anti-hsa-miR-320a antisense complementary to hsa-miR-320a.

10. The method according to claim 8 or 9, **characterized in that**, primer pairs which can express the sequence fragment of anti-hsa-miR-320a antisense complementary to hsa-miR-320a are shown as SEQ ID NO.4 and SEQ ID NO.5;
the expression vector is adeno-associated virus expression vector pAAV-D(+).

11. Biomarker for the diagnosing and/ or early warning diabetes, **characterized in that**, comprises sequence fragment containing hsa-miR-320a; the hsa-miR-320a is shown as SEQ ID No. 1.

12. The biomarker according to claim 11, **characterized in that**, comprises said hsamiR-320a.

13. The biomarker according to claim 11 or 12, **characterized in that**, the biomarker is said hsa-miR-320a.

14. A kit for diagnosing and/or early warning diabetes, **characterized in that**, comprises reagent for quantitative detection of biomarker according to any of claims 11-13.

15. The kit according to claim 14, **characterized in that**, comprises reagent for quantitative detection of hsa-miR-320a.

16. The kit according to claim 15, **characterized in that**, the reagent for quantitative detection of hsa-miR-320a comprises a specific primer pairs for hsa-miR-320a.

17. The kit according to claim 16, **characterized in that**, the specific primer pairs is commercially-available MIRQ0000510-1-1.

18. The kit according to claim 15 or 16, **characterized in that**, the regents for quantitative detection of hsa-miR-320a also comprises reverse transcription reagents;
and / or reverse transcription PCR reagents;
preferably, the reverse transcription reagent comprises:
RT Primer Mix, 2×TS reaction buffer, RNase free H₂O, TS enzyme;further preferably, the reverse transcription PCR reagent comprises: 2×SYBR Green Mix, RNase free H₂O.

19. The kit according to any of claims 14-18, **characterized in that**, the quantitative detection refers to fluorescent quantitative PCR detection;
most preferably, the kit comprises miR-320a RT-primers, miR-320a real-time PCR forward primers, miR-320areal-time PCR reverse primer, SYBR Green I, TS reaction enzyme, TS reaction buffer and DEPC ddH₂O.

20. Use of the biomarker according to any of claims 11-13 in preparing reagent for diagnosing diabetes.
